# FASCICULE DE BREVET EUROPEEN

(11) **EP 3 019 032 B1**
(45) Date de publication et mention de la délivrance du brevet: **29.03.2017**
(21) Numéro de dépôt: 14750547.3
(22) Date de dépôt: 25.06.2014
(51) Int. Cl.: A23L 17/60, A23K 10/00, A23K 30/15, A23K 40/00, A23L 29/00, A61K 36/05, C12N 1/12

(54) **PROCEDE DE PRODUCTION DE BIOMASSE DE MICROALGUES DE QUALITE SENSORIELLE OPTIMISEE**
VERFAHREN ZUR HERSTELLUNG EINER MIKROALGENBIOMASSE MIT OPTIMIERTER SENSORISCHER QUALITÄT
METHOD FOR THE PRODUCTION OF A MICROALGAL BIOMASS OF OPTIMISED SENSORY QUALITY

(30) Priorité: 26.06.2013 FR 1356110
(43) Date de publication de la demande: 18.05.2016
(73) Titulaire: Roquette Frères, 62136 Lestrem (FR)
(72) Inventeur: DRUON, Amandine, F-59000 Lille (FR); PATINIER, Samuel, F-59890 Quesnoy-sur-Deule (FR)
(74) Mandataire: Cabinet Becker et Associés
(86) Numéro de dépôt international: PCT/FR2014/051588
(87) Numéro de publication internationale: WO 2014/207376

(56) Documents cités:
- WO-A1-2010/120923
- WO-A2-2010/045368
- US-A1- 2010 303 989

## Description

La présente invention est relative à un nouveau procédé de production d'une biomasse de microalgues du genre *Chlorella* qui permet la préparation d'une farine présentant un profil sensoriel optimisé. La présente invention autorise alors l'incorporation de cette farine de microalgues dans des formulations alimentaires sans génération d'arômes indésirables.

### Présentation de l'état de l'art

Ne nécessitant historiquement « que de l'eau et de la lumière du soleil » pour croître, les algues sont considérées depuis longtemps comme une source de nourriture.

Il existe plusieurs espèces d'algues pouvant être utilisées en alimentation, la plupart étant des "macro-algues" comme le varech, la laitue de mer (*Ulva lactuca*) et des algues rouges de type *Porphyra* (cultivée au Japon) ou « dulse » (*Palmaria palmata*)*.*

Cependant, outre ces macroalgues, il y a aussi d'autres sources d'algues représentées par les « microalgues », c'est à dire des algues microscopiques unicellulaires photosynthétiques ou non, d'origine marine ou non, cultivées pour leurs applications dans les biocarburants ou l'alimentation.

Par exemple, la spiruline (*Arthrospira platensis*) est cultivée dans des lagunes ouvertes (par phototrophie) pour une utilisation comme complément alimentaire ou incorporée en petites quantités dans la confiserie ou les boissons (généralement moins de 0,5% poids / poids).

D'autres microalgues riches en lipides, y compris certaines espèces de type *Chlorella*, sont aussi très populaires dans les pays asiatiques comme compléments alimentaires (mention est faite de microalgues du genre *Crypthecodinium* ou *Schizochytrium* producteurs d'acides gras omega 3).

La production et l'utilisation de la farine de microalgues de type *Chlorella* sont par exemple décrits dans les documents WO 2010/120923 et WO 2010/045368.

La fraction huile de la farine de microalgues, qui peut être composée essentiellement d'huiles mono-insaturées, peut offrir des avantages nutritionnels et de santé par rapport aux huiles saturées, hydrogénées et polyinsaturés souvent trouvées dans les produits alimentaires conventionnels.

Lorsque l'on souhaite fabriquer industriellement des poudres de farine de microalgues à partir de leur biomasse, d'importantes difficultés demeurent, non seulement du point de vue technologique mais également du point de vue du profil sensoriel des farines produites.

En effet, si des poudres d'algues par exemple fabriquées avec des algues cultivées photosynthétiquement dans des étangs extérieurs ou par photobioréacteurs sont disponibles dans le commerce, elles ont une couleur vert foncée (liée à la chlorophylle) et un goût fort, désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines.

Par ailleurs, il est connu que certaines espèces d'algues bleues produisent naturellement des molécules chimiques odorantes telles que la géosmine (trans-1,10-diméthyl-trans-9-decalol) ou le MIB (2-méthylisoborneol), générant des odeurs terreuses ou de moisi.

Quant aux chlorelles, le descripteur communément admis dans ce domaine est le goût de « thé vert », un peu semblable à d'autres poudres végétales vertes telles que l'orge vert en poudre ou le blé vert en poudre, goût attribué à sa forte teneur en chlorophylle.

Leur saveur n'est habituellement masquée que lorsqu'elles sont mélangées avec des légumes à saveur forte ou des jus d'agrumes.

Il existe donc toujours un besoin non satisfait de disposer d'un procédé de préparation de la biomasse de microalgues du genre *Chlorella* de qualité organoleptique convenable permettant l'utilisation de la farine préparée à partir de celles-ci dans des produits alimentaires plus nombreux et diversifiés. Par ailleurs, toujours dans un esprit d'optimisation industrielle, un procédé fournissant de manière reproductible des microalgues du genre *Chlorella* de qualité organoleptique reproductible serait très avantageux.

### Objet de l'invention

Pour concevoir le procédé de l'invention, la société Demanderesse a tout d'abord choisi de former un panel sensoriel pour évaluer les propriétés sensorielles de différents lots de farine de biomasse de *Chlorella protothecoides.* La qualification sensorielle de lots de production autorise alors l'identification des étapes clefs du procédé qui permettront la production de farine de biomasse de microalgues de qualité organoleptique conforme aux attentes, et ce de manière reproductible.

Dans sa conduite de production de la biomasse de microalgues par fermentation en conditions hétérotrophiques et en absence de lumière, comme il sera exemplifié ci-après, la société Demanderesse a alors fait varier les différents paramètres de fermentation et de traitement de la biomasse afin de générer ces différents lots. La société Demanderesse a finalement réussi à mettre en évidence une corrélation entre la note sensorielle donnée par le panel sensoriel à chaque lot produit et certaines des conditions de conduite du procédé de production desdits lots.

Cette corrélation a alors permis à la société Demanderesse de sélectionner les paramètres de conduite de la fermentation et de traitement de la biomasse qui, pris seul ou en combinaison, garantissent la production de biomasse de *Chlorella* présentant un profil sensoriel optimisé.

La société Demanderesse a alors proposé un procédé de production de conditionnement d'une biomasse de microalgues du genre *Chlorella,* de préférence *Chlorella protothecoides*, pour la préparation d'une farine présentant un profil sensoriel optimisé.

La présente invention est donc relative à un procédé de conditionnement d'une biomasse de microalgues du genre *Chlorella*, de préférence *Chlorella protothecoides*, produites en conditions hétérotrophiques et en absence de lumière pour la préparation d'une farine présentant un profil sensoriel optimisé, le procédé de conditionnement étant caractérisé en ce qu'il comprend les étapes de :
- collecte de la biomasse directement en fin de fermentation, et stockage pendant moins de 8 heures avant son conditionnement,
- traitement thermique Haute Température / Temps Court (HTST) de la biomasse ainsi récupérée et stockée, pendant 30 secondes à 1 min 30, à une température inférieure à 70 °C, et
- lavage de la biomasse avec au maximum 3 volumes d'eau par volume de biomasse.

De préférence, la durée de stockage de la biomasse avant son conditionnement et son broyage est de moins de 3 heures, de préférence de moins de 1 heure.

De préférence, le traitement thermique HTST est mené pendant 1 minute à une température comprise entre 60 et 68°C, de préférence, 65°C ± 2°C, en particulier 65°C.

De préférence, la biomasse est lavée avec un volume d'eau par volume de biomasse.

Dans un mode de réalisation préféré, le traitement HTST est réalisé avant l'étape de lavage de la biomasse.

Dans un mode de réalisation préféré, la biomasse conditionnée a été obtenue par une fermentation de la microalgue du genre Chlorella, de préférence *Chlorella protothecoides*, à un pH initial entre 6,5 et 7, de préférence 6,8, et avec la régulation du pH de fermentation à une valeur comprise entre 6,5 et 7, de préférence à une valeur de 6,8.

La présente invention est également relative à un procédé de production d'une biomasse de microalgues du genre *Chlorella*, de préférence *Chlorella protothecoides*, pour la préparation d'une farine présentant un profil sensoriel optimisé, comprenant :
- la production d'une biomasse par fermentation de microalgues du genre *Chlorella*, de préférence *Chlorella protothecoides*, en conditions hétérotrophiques et en l'absence de lumière, le pH initial de la fermentation et la régulation du pH pendant fermentation étant fixée à une valeur comprise entre 6,5 et 7, de préférence à une valeur de 6,8 ; et
- le conditionnement de la biomasse par une méthode telle que décrite dans le présent document.

Enfin, présente invention est également relative à un procédé de préparation d'une farine de microalgues du genre *Chlorella*, de préférence *Chlorella protothecoides*, présentant un profil sensoriel optimisé, comprenant :
- le conditionnement de la biomasse par une méthode telle que décrite dans le présent document ;
- le broyage de la biomasse conditionnée ; et
- le séchage de la biomasse broyée.

Facultativement, le procédé comprend en outre, préalablement au conditionnement, la production d'une biomasse par fermentation de microalgues du genre *Chlorella*, de préférence *Chlorella protothecoides*, en conditions hétérotrophiques et en l'absence de lumière, le pH initial de la fermentation et la régulation du pH pendant fermentation étant fixée à une valeur comprise entre 6,5 et 7, de préférence à une valeur de 6,8.

### Description détaillée de l'invention

Au sens de l'invention, une farine de microalgues présente un « profil sensoriel optimisé », lorsque son évaluation par un panel sensoriel en formulation alimentaire ou de dégustation (par exemple, crème glacée ou recette de dégustation telle que décrite ici) conclut à l'absence de défauts qui altèrent la qualité organoleptique desdites formulations alimentaires contenant cette farine de microalgues.

Ces défauts (terme anglo-saxon de « off-notes ») peuvent être associés à la présence de molécules spécifiques odorantes et/ou aromatiques indésirables qui se caractérisent par un seuil de perception correspondant à la valeur minimale du stimulus sensoriel nécessaire à l'éveil d'une sensation.

Le « profil sensoriel optimisé » est alors traduit par un panel sensoriel par l'obtention des meilleurs scores sur une échelle d'évaluation des 4 critères sensoriels (aspect, texture, saveurs et flaveurs).

Au sens de la présente invention, le terme « farine de microalgues » doit être compris dans son interprétation la plus large et comme désignant par exemple, une composition comprenant une pluralité de particules de biomasse de microalgues. La biomasse de microalgues est dérivée de cellules de microalgues, qui peuvent être entières ou lysées, ou un mélange de cellules entières et lysées.

Un certain nombre de documents de l'état de l'art, comme la demande de brevet internationale WO 2010/120923, décrivent des méthodes de fabrication et d'utilisation en alimentation de la biomasse de microalgues de *Chlorella.*

Les microalgues dont il est question dans la présente invention sont des microalgues du genre *Chlorella*, plus particulièrement *Chlorella protothecoides*, plus particulièrement encore des *Chlorella* privées de pigments chlorophylliens, par toute méthode connue en soi de l'homme du métier (soit par le fait que la culture est réalisée à l'obscurité dans certaines conditions opératoires bien connues de l'homme du métier, soit parce que la souche a été mutée de manière à ne plus produire ces pigments).

Le procédé fermentaire décrit dans cette demande de brevet WO 2010/120923 permet ainsi la production d'un certain nombre de farines de microalgues de qualité sensorielle variable, si l'on fait varier les conditions de fermentation et de traitement de la biomasse produite.

La société Demanderesse a ainsi choisi de faire varier et d'analyser l'impact des paramètres suivants :
- pH initial du milieu de fermentation,
- pH encours de fermentation,
- acidification lors de la récolte du moût de fermentation,
- traitement thermique de la biomasse, (traitement dit HTST)
- lavage de la biomasse,
- broyage de la biomasse,
- ajustement du pH,
- pasteurisation
- séchage, et
- stockage de la farine ainsi obtenue.

Comme il a été exemplifié ci-après, les étapes clefs du procédé de conditionnement de la biomasse de manière à optimiser le profil sensoriel de farines de microalgues sont les suivantes :
- collecte de la biomasse directement en fin de fermentation, et stockage pendant moins de 8 heures avant son conditionnement précédent son broyage,
- traitement HTST de la biomasse ainsi récupérée et stockée, mené entre 30 secondes et 1 min 30, à une température inférieure à 70 °C,
- lavage de la biomasse traitée par HTST avec au maximum 3 volumes d'eau par volume de biomasse.

Ainsi, la biomasse est collectée le plus rapidement possible pour subir les étapes ultérieures de traitement thermiques et/ou de lavage. Il a été déterminé que le stockage doit être le plus court possible. De préférence, le stockage dure moins de 8, 7, 6, 5, 4, 3, 2 ou 1 heures. De préférence, la durée de stockage de la biomasse avant son conditionnement et son broyage est de moins de 3 heures, de préférence de moins de 1 heure. Idéalement, l'étape de stockage est absente et la biomasse collectée est directement soumise aux étapes ultérieures de traitement thermiques et/ou de lavage.

Il a été également déterminé que le traitement thermique HTST avait également un impact sur le profil sensoriel et donc la qualité organoleptique de la farine de microalgues. Ainsi, la température est de préférence inférieure ou égale à 70 °C et supérieure à 50 °C. Elle peut être comprise entre 55 et 70°C, de préférence entre 60 et 68 °C, de préférence de 65 °C. Le temps de traitement est de préférence de 1 minute.

Il a en outre été montré que le lavage pouvait être optimisé tout en améliorant le profil sensoriel et donc la qualité organoleptique de la farine de microalgues. Ainsi, de préférence, la biomasse est lavée avec 3, 2,5, 2, 1,5 ou 1 volume d'eau pour un volume de biomasse. Dans un mode de réalisation, on utilisera un volume d'eau pour un volume de biomasse.

Enfin, il a été montré que l'ordre des étapes de conditionnement avait un impact sur le profil sensoriel et donc la qualité organoleptique de la farine de microalgues. Notamment, il est préférable de réaliser le traitement thermique HTST avant l'étape de lavage de la biomasse.

La biomasse de microalgues conditionnée est une biomasse préparée par fermentation en conditions hétérotrophiques et en absence de lumière d'une microalgue du genre *Chlorella*, de préférence *Chlorella protothecoides*.

Facultativement, les microalgues utilisées peuvent être choisies, et manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora* et *Prototheca moriformis.*

De manière préférée, les microalgues utilisées selon l'invention appartiennent à l'espèce *Chlorella protothecoides.* Selon ce mode préféré, les algues destinées à la production de la farine de microalgues possèdent le statut GRAS. Le concept GRAS (Generally Recognized As Safe) créé en 1958 par la Food and Drug Administration (FDA) permet la régulation de substances ou extraits ajoutés aux aliments et qui sont considérés comme sans danger par un panel d'expert.

Les conditions de fermentation sont bien connues de l'homme du métier. Les conditions appropriées de culture à utiliser sont notamment décrites dans l'article d'Ikuro Shihira-Ishikawa et Eiji Hase, « Nutritional Control of Cell Pigmentation in Chlorella protothecoides with special reference to the degeneration of chloroplast induced by glucose », Plant and Cell Physiology, 5, 1964.

Cet article décrit notamment que tous les grades de couleur peuvent être produits par *Chlorella protothecoides* (incolore, jaune, vert jaunâtre et vert), en faisant varier les sources et les ratios d'azote et de carbone. En particulier, des cellules « délavées » et « incolores » sont obtenues en utilisant des milieux de culture riches en glucose et pauvres en azote.

La distinction entre cellules incolores et cellules jaunes est faite dans cet article. De plus, les cellules délavées cultivées en excès de glucose et en azote limité possèdent de fort taux de croissance. De plus, ces cellules contiennent de fortes quantités de lipides.

D'autres articles, tel que celui de Han Xu, Xiaoling Miao, Qingyu Wu , « High quality biodiesel production from a microalga Chlorella protothecoides by heterotrophic growth in fermenters », Journal of Biotechnoloy, 126, (2006), 499-507 décrivent que des conditions de culture hétérotrophiques, c'est-à-dire en absence de lumière permettent d'obtenir une biomasse élevée avec une teneur élevée en lipides dans les cellules de microalgues.

Les milieux de croissance solides et liquides sont généralement disponibles dans la littérature, et les recommandations pour la préparation des milieux particuliers qui conviennent à une grande variété de souches de microorganismes peuvent être trouvées, par exemple, en ligne à www.utex.org/, un site maintenu par l'Université du Texas à Austin pour sa collection de culture d'algues (UTEX).

Au vu de ses connaissances générales et de l'état de l'art précité, l'homme du métier chargé de cultiver les cellules de microalgues sera tout à fait capable d'adapter les conditions de culture afin d'obtenir une biomasse adaptée, de préférence riche en lipides.

Notamment, le protocole de fermentation peut être défini sur la base de celui décrit en toute généralité dans la demande de brevet WO 2010/120923.

Selon la présente invention, les microalgues sont cultivées en milieu liquide pour produire la biomasse en tant que telle.

La production de biomasse est réalisée en fermenteurs (ou bioréacteurs). Les exemples spécifiques de bioréacteurs, les conditions de culture, et la croissance hétérotrophe et les méthodes de propagation peuvent être combinés de toute manière appropriée pour améliorer l'efficacité de la croissance microbienne et des lipides et / ou de production de protéines.

Dans un mode de réalisation particulier, la fermentation est réalisée en mode fed-batch avec un débit de glucose ajusté de sorte à maintenir une concentration de glucose résiduel de 3 à 10 g/l.

Pendant la phase d'alimentation en glucose, la teneur en azote dans le milieu de culture est de préférence limitée pour permettre l'accumulation de lipides à hauteur de 30, 40, 50 ou 60 %. La température de fermentation est maintenue à une température adaptée, de préférence ente 25 et 35 °C, en particulier 28°C. L'oxygène dissous est de préférence maintenu à un minimum de 30 % en contrôlant l'aération, la contre pression et l'agitation du fermenteur.

Dans un mode de réalisation préféré, il a été montré que le pH lors de la fermentation avait un impact sur la qualité organoleptique du produit final. Ainsi, le pH initial de la fermentation est fixé entre 6,5 et 7, de préférence à une valeur de 6,8, et il est ensuite régulé pendant fermentation à une valeur comprise entre 6,5 et 7, de préférence à une valeur de 6,8. La durée de la fermentation de production est de 3 à 6 jours, par exemple de 4 à 5 jours.

De préférence, la biomasse obtenue a une concentration comprise entre 130g/l et 250 g/l, avec une teneur en lipides d'environ 50% en poids sec, une teneur en fibres de 10% à 50% en poids sec, une teneur en protéines de 2% à 15 % en poids sec, et une teneur en sucres inférieure à 10% en poids.

Ensuite, la biomasse obtenue en fin de fermentation est récoltée du milieu de fermentation et soumise au procédé de conditionnement tel que décrit ci-dessus.

Après le conditionnement, la biomasse est transformée en farine de microalgues. Les principales étapes de préparation de la farine de microalgues comprennent notamment le broyage, l'homogénéisation et le séchage.

La farine de microalgues peut être préparée à partir de la biomasse de microalgues concentrée qui a été mécaniquement lysée et homogénéisée, l'homogénat étant ensuite atomisé ou flash-séché.

Les cellules de la biomasse utilisées pour la production de farine de microalgues sont de préférence lysées pour libérer leur huile ou lipides. Les parois cellulaires et les composants intracellulaires sont broyés ou réduits, par exemple grâce à un homogénéisateur, en particules ou débris cellulaires non agglomérés. De préférence, les particules résultantes possèdent une taille moyenne inférieure à 500 µm, 100 µm ou même 10 µm ou moins.

Les cellules lysées peuvent également être séchées. Par exemple, un disrupteur à pression peut être utilisé pour pomper une suspension contenant les cellules à travers un orifice restreint pour lyser les cellules. Une pression élevée (jusqu'à 1500 bars) est appliquée, suivie d'une expansion instantanée à travers une buse. Le cassage des cellules peut être réalisé par trois mécanismes différents : empiètement sur la vanne, cisaillement élevé du liquide dans l'orifice, et chute de pression soudaine en sortie, provoquant une explosion de la cellule. Un homogénéisateur NIRO (GEA NIRO SOAVI) (ou tout autre homogénéisateur haute pression) peut être utilisé pour casser des cellules.Ce traitement de la biomasse algale sous haute pression (environ 1500 bars) lyse généralement plus de 90 % des cellules et réduit la taille des particules à moins de 5 microns. De préférence, la pression appliquée est de 900 bars à 1200 bars, en particulier 1100 bars.

En outre, et afin d'augmenter le pourcentage de cellules lysées, la biomasse de microalgues peut subir un double traitement à haute pression, voire plus (triple traitement, ..). De préférence, une double homogénéisation est utilisée afin d'augmenter le pourcentage de cellules lysées supérieur à 50%, supérieur à 75% ou supérieur à 90%. Le pourcentage de cellules lysées d'environ 95% a été observé grâce à ce double traitement.

La lyse des cellules de microalgues est optionnelle mais préférée lorsqu'une farine riche en lipides (notamment; supérieure à 10%) est souhaitée. Ainsi, la farine de microalgues peut éventuellement être sous la forme de cellules non lysées.

De préférence, au moins une lyse partielle est souhaitée, c'est-à-dire que la farine de microalgues est sous la forme de cellules partiellement lysées et contient de 25% à 75% de cellules lysées. De préférence, une lyse maximale voire totale est souhaitée, c'est-à-dire que la farine de microalgues est sous la forme de cellules fortement voire totalement lysées et contient 85% ou plus des cellules lysées, de préférence 90% ou plus. Ainsi, la farine de microalgues est susceptible d'être sous une forme non broyée jusqu'à une forme extrêmement broyée avec des taux de broyage supérieurs à 95%. Des exemples spécifiques portent sur des farines de microalgues présentant des taux de broyage de 50%, 85% ou 95 % de lyse des cellules, de préférence 85% ou 95 %.

De manière alternative, un broyeur à billes peut être utilisé. Dans ce type de broyeur, les cellules sont agitées en suspension avec de petites particules abrasives. Le cassage des cellules est provoqué par les forces de cisaillement, le broyage entre les billes, et les collisions avec des billes. En fait, ces billes cassent les cellules pour en libérer le contenu cellulaire. La description d'un broyeur à billes approprié est par exemple faite dans le brevet US 5.330.913.

On obtient ainsi une suspension de particules, optionnellement de plus petite taille que les cellules d'origine sous la forme d'une émulsion « huile dans eau ».

Cette émulsion peut ensuite être atomisée et l'eau est éliminée, laissant une poudre sèche contenant les débris cellulaires et les lipides. Après séchage, la teneur en eau ou l'humidité de la poudre est généralement inférieure à 10 %, préférentiellement inférieure à 5% et de façon plus préférée inférieure à 3% en poids.

De préférence, la farine de microalgues est préparée sous forme de granules. Les granules de farine de microalgues sont susceptibles d'être obtenus par un procédé d'atomisation particulier, qui met en oeuvre des buses de pulvérisation à haute pression dans une tour à flux parallèles qui dirige les particules vers une bande mobile située dans le bas de la tour. La matière est ensuite transportée comme une couche poreuse à travers des zones de post-séchage et de refroidissement, qui lui donnent une structure craquante, comme celle d'un gâteau qui se brise à l'extrémité de la bande. La matière est ensuite mise en oeuvre pour obtenir la taille de particules souhaitée. Pour procéder à la granulation de la farine d'algues, en suivant ce principe d'atomisation, on peut employer par exemple un atomiseur FILTERMAT™ commercialisé par la société GEA NIRO ou un système de séchage TETRA MAGNA PROLAC DRYER™ commercialisé par la société TETRA PAK.

Dans un mode de réalisation préféré, suite au procédé de conditionnement, le procédé de préparation des granules de farine de microalgues peut comprendre les étapes suivantes :
1) préparer une émulsion de farine de microalgues à une matière sèche comprise entre 15 et 40 % en poids sec,
2) introduire cette émulsion dans un homogénéisateur haute pression. Cette homogénéisation haute pression de l'émulsion peut être accomplie dans un dispositif à deux étages, par exemple un homogénéisateur GAULIN commercialisé par la société APV, avec une pression de 100 à 250 bars au premier étage, et 10 à 60 bars au second étage.
3) la pulvériser dans un atomiseur vertical équipé d'une bande mobile à sa base, et d'une buse haute pression à sa partie supérieure, tout en réglant :
   a) la pression appliquée au niveau des buses de pulvérisation à des valeurs de plus de 100 bars, de préférence entre 100 et de 200 bars, et de préférence encore entre 160 et 170 bars,
   b) la température d'entrée comprise entre 150°C et250°C, de préférence entre 180°C et 200°C, et
   c) la température de sortie dans cette zone d'atomisation comprise entre 60°C et 120°C, de préférence entre 60°C et 110°C et de préférence encore entre 60°C et 80°C,
4) régler les températures d'entrée de la zone de séchage sur la bande mobile entre 40°C et 90°C, de préférence entre 60°C et 90°C, et la température de sortie entre 40°C et 80°C, et régler les températures d'entrée de la zone de refroidissement à une température entre 10°C et 40°C, de préférence entre 10°C et 25°C, et la température de sortie entre 20°C et 80°C, de préférence entre 20°C et 60°C,
5) collecter les granules de farine de microalgues ainsi obtenus.

Selon l'invention, la biomasse extraite du milieu de fermentation par tout moyen connu de l'homme du métier est ensuite :
- concentrée (par exemple par centrifugation)
- optionnellement conservée par l'addition de conservateurs standards (benzoate de sodium et sorbate de potassium par exemple),
- broyée cellulairement.

L'invention sera mieux comprise à l'aide des exemples qui suivent, lesquels se veulent illustratifs et non limitatifs.

### EXEMPLES

### Exemple 1. Préparation des différents lots de farine de biomasse de Chlorella protothecoides

### A. Descriptif du protocole standard : de la production de la biomasse à celle de la farine

### 1. Fermentation

Le protocole de fermentation est adapté de celui décrit en toute généralité dans la demande de brevet WO 2010/120923.

Le fermenteur de production est inoculé avec une préculture de *Chlorella protothecoides.* Le volume après inoculation atteint 9 000 l.

La source de carbone employée est un sirop de glucose 55 % poids/poids stérilisé à 130°C pendant 3 minutes.

La fermentation est conduite en mode fed-batch avec un débit de glucose ajusté de sorte à maintenir une concentration de glucose résiduel de 3 à 10 g/l.

La durée de la fermentation de production est de 4 à 5 jours.

En fin de fermentation, la concentration cellulaire atteint 185 g/l.

Pendant la phase d'alimentation en glucose, la teneur en azote dans le milieu de culture est limitée pour permettre l'accumulation de lipides à hauteur de 50 %.

La température de fermentation est maintenue à 28°C.

Le pH de fermentation avant inoculation est ajusté à 6,8 puis est régulé sur cette même valeur pendant la fermentation.

L'oxygène dissous est maintenu à un minimum de 30 % en contrôlant l'aération, la contre pression et l'agitation du fermenteur.

### 2. Conditionnement de la biomasse

Le moût de fermentation est traité thermiquement sur une zone Haute Température / Temps Court (acronyme anglo-saxon : « HTST ») pendant 1 min à 75°C et refroidi à 6°C.

La biomasse est alors lavée à l'eau potable décarbonatée avec un ratio de dilution de 6 volumes d'eau pour 1 volume de biomasse et concentrée par centrifugation en utilisant une Alfa Laval FEUX 510.

La biomasse est alors acidifiée à pH 4 à l'acide phosphorique 75 % puis des conservateurs sont ajoutés (500 ppm benzoate de sodium / 1000ppm sorbate de potassium).

### 3. Broyage de la biomasse

La biomasse est alors broyée avec un broyeur à billes NETZSCH LME500 en utilisant des billes de silicate de zirconium de 0,5mm de diamètre.

Le taux de broyage ciblé est de 85 à 95%.

Le produit est maintenu refroidi tout au long de ce procédé au cours des phases de stockage et par refroidissement en ligne avec des échangeurs dédiés.

Des antioxydants sont ajoutés (150 ppm/sec d'acide ascorbique et 500ppm/sec d'un mélange de tocophérols) en prévention de dégradation par oxydation.

Le milieu est ajusté à pH 7 à la potasse.

### 4. Séchage de la farine

Le produit est alors pasteurisé à 77°C, pendant 3 minutes en ligne avec l'opération de séchage.

Cette dernière est effectuée sur un Filtermat FMD125 avec un cyclone. La pression des buses est de 160-170 bar.

### B. Définition du panel sensoriel et des descripteurs permettant l'évaluation de la qualité organoleptique des farines de microalgues obtenue à partir de la biomasse

Un ensemble de 14 personnes a donc été rassemblé pour évaluer les différents lots de biomasse produits à l'aide des descripteurs suivant :

| **Descripteurs** | | **Référence** |
|---|---|---|
| Aspect | Couleur (de clair à foncé) | |
| Texture | Nappant | lait entier + 5% crème |
| Saveurs | Sucré | Saccharose 1% |
| Flaveurs | Champignon | 100 g de champignons dans 100 ml d'eau froide / dilution X 4 |
| | Céréales | Solution Ebly 10% |
| | Beurre / produit laitier | |
| | Huile rance | Huile oxydée 1,5% |
| | Arrière goût végétal | Farine de microalgues très inacceptable |

La société Demanderesse a ensuite trouvé que la matrice de dégustation est avantageusement construite à partir de la formule suivante :
- 7 % de Farine de microalgues
- 1 % sucre cristal
- 0,25 % arôme vanille ménager
- 91,75 % lait écrémé.

Le mélange est homogénéisé avec un mixeur à immersion jusqu'à obtention d'un mélange homogène (environ 20 secondes), puis il est chauffé à 75°C pendant 5 minutes au bain marie.

A chaque séance de dégustation, 4 à 5 produits sont évalués sur chaque descripteur en comparaison d'un lot de référence de farine de microalgues 1.

Tous les produits sont évalués les uns après les autres, sur des échelles allant de 1 à 9 de la manière suivante :

| | |
|---|---|
| Valeur de 1 : | le descripteur évalué n'est pas présent dans le produit |
| Valeur de 5 : | le descripteur évalué est présent dans le produit exactement de la même façon que sur le produit de référence 1. |
| Valeur de 9 : | le descripteur évalué est très présent dans le produit. |

Le lot de référence 1 est une farine de microalgues conforme au sens où elle présente le profil sensoriel "satisfaisant" tous ces descripteurs.

De préférence, le lot de référence 1 n'est pas à considérer comme la farine de microalgues présentant le profil sensoriel optimisé : il s'agit d'une farine de microalgues perçue par le panel sensoriel comme "satisfaisant", notamment présentant une note de 5 sur tous les descripteurs testés.

Les autres lots de farine de microalgues seront donc classées par le panel sensoriel de part et d'autres de ce lot de référence 1.

De préférence, un lot de référence 2 considéré comme « très inacceptable » car ne satisfaisant pas les descripteurs relatifs aux notes aromatiques, notamment de Saveurs et de Flaveurs est également inclus. Ce lot est donc le plus éloigné possible du lot de référence 1.

Des analyses de variances (ANOVA) sont réalisées pour évaluer le pouvoir discriminant des descripteurs (descripteurs dont la p-value associée au test de Fisher - ANOVA de type 3 - est inférieure à 0,20 pour l'effet Farine dans le modèle descripteur ∼ Farine + juge).

L'effet Farine s'interprète comme le pouvoir discriminant des descripteurs : s'il n'y a pas d'effet (Probabilité Critique > 0,20), les farines n'ont pas été discriminées selon ce critère. Plus la probabilité critique est petite, plus le descripteur est discriminant.

Une Analyse en Composantes Principales (ACP) est ensuite réalisée afin d'obtenir une cartographie sensorielle des farines, et une représentation simultanée de toutes les farines sur tous les descripteurs.

### Logiciel de traitement des données

Les analyses ont été réalisées à l'aide du logiciel R (en vente libre) :
R version 2.14.1 (2011-12-22)
Copyright (C) 2011 The R Foundation for Statistical Computing
ISBN 3-900051-07-0
Platform: i386-pc-mingw32/i386 (32-bit)

Le logiciel est un environnement de travail qui nécessite le chargement de modules contenant les fonctions de calcul.

Les modules utilisés dans cette étude sont les suivants :
- Pour l'ACP : Package *FactoMineR* version 1.19
- Pour l'ANOVA : Package car version 2.0-12

### C. Impact du pH de la fermentation

Les conditions de pH de fermentation sont classiquement définies dans le protocole standard en partant du postulat selon lequel le pH de fermentation doit être fixé à une valeur de 6,8 (pH de l'optimum de croissance connu de l'homme du métier pour des microalgues du genre *Chlorella protothecoides*), mais sans que l'impact de cette valeur de pH sur les qualités organoleptiques des farines de microalgues ne soit ni étudié ni établi.

Deux séries de lots de farine sont donc produites à partir de biomasse préparées à deux conditions de pH : neutres (6,8) et acides (5,2). Cette valeur de 5,2 a été choisie de manière à tenir compte des contraintes bactériologiques (un pH acide étant peu favorable à la croissance de bactéries contaminantes).

Le tableau I suivant présente les références des lots produits à ces deux valeurs de pH.

**Tableau I.**

| lots | pH |
|---|---|
| lot 21 | 6,8 |
| lot 23 | 6,8 |
| lot 24 | 5,2 |
| lot 31 | 6,8 |
| lot 53 | 5,2 |
| lot 61 | 5,2 |
| lot 111 | 6,8 |
| lot 131 | 6,8 |

Chacun de ces lots est ensuite évalué par le panel sensoriel selon les descripteurs présentés ci-avant.

Les 8 différents lots (lot 21, lot 23, lot 24, lot 31, lot 53, lot 61, lot 111 et lot 131) ont été analysés selon la méthode décrite ci-dessus.

On présente ici deux exemples sur les descripteurs "beurre / produits laitiers" et "arrière-goût végétal".

**"arrière goût végétal" : Tableau d'analyse de variance**

| | Df | Somme Sq | Moyenne Sq | Valeur F | **Pr(>F)** |
|---|---|---|---|---|---|
| **Farine** | 9 | 109,693 | 12,1881 | 18,2423 | **< 2e-16** |
| Juge | 13 | 18,732 | 1,4409 | 2,1566 | |
| 0,01298 | | | | | |
| Residus | 185 | 123.603 | 0.6681 | | |
| --- | | | | | |

**"beurre / produits laitiers" : Tableau d'analyse de variance**

| | Df | Somme Sq | Moyenne Sq | Valeur F | **Pr(>F)** |
|---|---|---|---|---|---|
| **Farine** | 9 | 8,292 | 0,92131 | 1,4530 | |
| **0,1699** | | | | | |
| Juge | 13 | 8,235 | 0,63347 | 0,9991 | |
| 0,4547 | | | | | |
| Residus | 160 | 101,451 | 0,63407 | | |
| --- | | | | | |

Il apparait que les probabilités critiques associées à l'effet Farine pour les 2 descripteurs étudiés sont inférieures à 0,2 : les 2 descripteurs sont donc discriminants. La probabilité critique est plus petite sur le descripteur « arrière-goût végétal » que sur le descripteur « beurre / produits laitiers », ce qui signifie qu'on observe plus de différence entre les Farines sur le premier critère que sur le deuxième.

Le tableau II suivant récapitule les probabilités critiques obtenues pour les effets Farine et juge pour tous les descripteurs.

**Tableau II**

| | Farine | Juge |
|---|---|---|
| Couleur | 1,62E-31 | 1,16^{E}-05 |
| arrière-goût végétal | 1,60E-21 | 1,30^{E}-02 |
| Goût huile rance | 4,00E-06 | 9,00^{E}-04 |
| Nappant | 1,48E-05 | 1,63^{E}-02 |
| Céréales | 4,05E-04 | 1,94^{E}-07 |
| Champignons | 1,37E-03 | 5,66^{E}-05 |
| Sucré | 3,23E-03 | 4,02^{E}-04 |
| Produits laitiers | 1,70E-01 | 4,55^{E}-01 |

Tous les descripteurs sont discriminants, on les garde tous pour établir l'ACP.

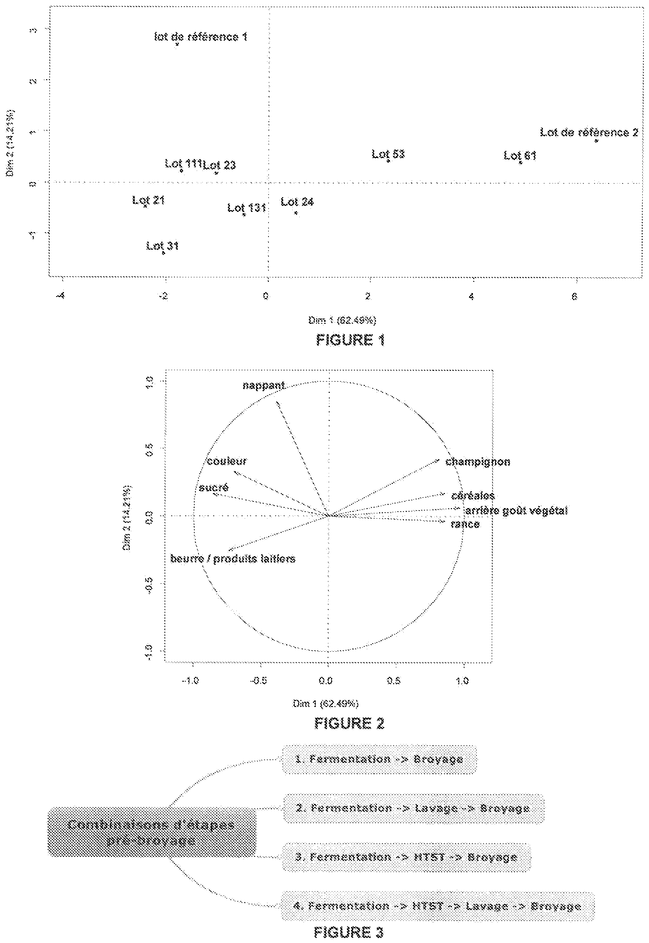

Puisque l'aromatique est un critère essentiel des farines, l'ACP a été réalisée sur les descripteurs relatifs aux arômes uniquement (champignon, céréales, arrière-goût végétal, produit laitier, rance). La représentation graphique de cette ACP est la Figures 1 et 2.

Le premier axe de l'ACP résumant plus de 75% de l'information, ce sont les coordonnées des produits sur cet axe qui nous servent de « variable / classement ». Ce classement rend donc bien compte des distances sensorielles entre les produits.

Cette méthode permet d'établir un classement de la qualité organoleptique de différentes farines de microalgues, que l'on peut figurer comme suit :
Lot 111 > lot 31 > lot 21 > lot 23 > Lot référence 1 > lot 131 > lot 24 > lot 53 > lot 61 > lot réf 2, avec une séparation nette entre les lots 111, 31, 21, 23 et 131 d'une part, et les lots 24, 53, et 61 d'autre part.

D'un point de vue global, le panel a jugé les lots 111, 31, 21, 23 et 131 acceptables et les lots 24, 53, et 61 inacceptable.

Ces résultats illustrent donc bien l'impact du pH de fermentation sur la présence d'arrière-goût rédhibitoire pour l'acceptabilité du produit.

A pH 5,2, le profil sensoriel présente systématiquement un arrière-goût végétal, alors qu'à pH 6,8, le profil sensoriel est globalement plus neutre, sans arrière-goût végétal significatif.

En première lecture, il apparaît donc que le contrôle du pH de la fermentation à une valeur de 6,8 est un critère clef pour la préparation de farine de microalgues présentant un profil sensoriel convenable, voir optimisé (lot 111).

Cependant, étant donné la variabilité organoleptique des lots produits à pH 6,8, force est de constater que le pH n'est pas le seul paramètre responsable des effets observés.

### D. Mesure de l'impact des étapes de conditionnement de la biomasse avant son broyage sur la qualité organoleptique de la farine produite

On a étudié également l'influence des deux principales étapes de conditionnement (= pré-broyage) de la biomasse avant son broyage : le traitement thermique HTST et le lavage.

A partir de la même biomasse produite à pH 6,8 selon l'étape 1 du procédé standard décrit ci-dessus, les étapes de conditionnement de la biomasse ont été réalisées selon quatre combinaisons différentes (Figure 3).

Celles-ci ont permis la production de 4 lots : n° 1 à 4 :
- Combinaison n° 1 est le témoin sans traitement HTST ni lavage.
- Combinaison n°2 : lavage seul
- Combinaison n°3 : HTST seul
- Combinaison n°4 : HTST avant Lavage

4 lots de farine sont produites selon ces 4 combinaisons. La suite des étapes est commune à chaque série et permet de conditionner l'échantillon pour l'analyse sensorielle. Le Tableau III suivant présente la liste des descripteurs discriminants sur cet ensemble de produits (p-value inférieure à 0,2 sur l'effet Farine):

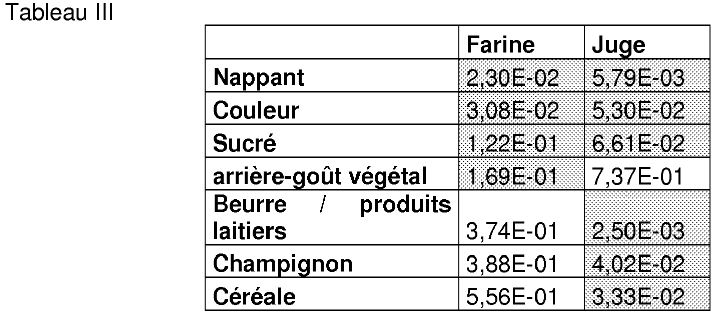

On réalise une Analyse en Composante Principale pour représenter les différences entre les différentes farines produites (en regard d'une farine sélectionnée comme Référence 1, c'est-à-dire, comme expliqué ci-dessus, farine de microalgues perçue par le panel sensoriel comme "satisfaisant", notamment présentant une note de 5 sur tous les descripteurs testés).

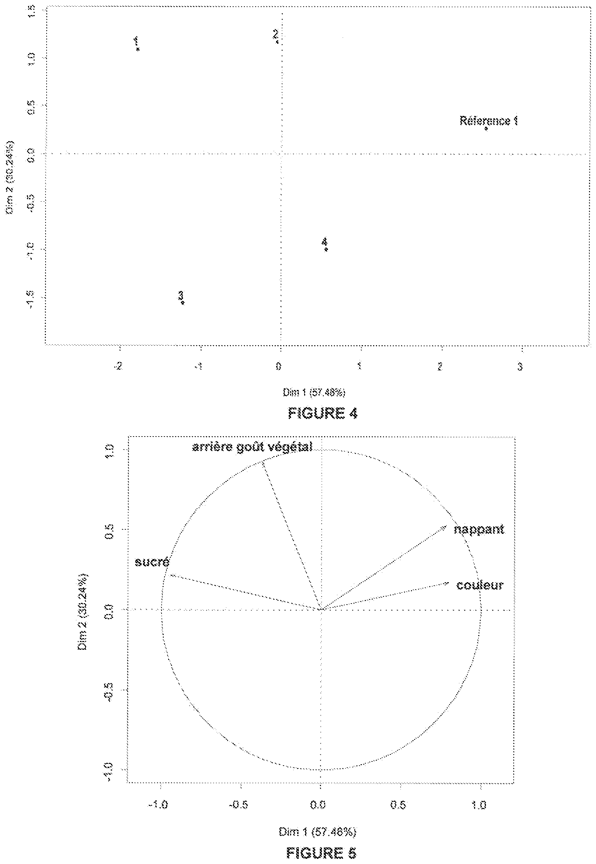

Les résultats sont présentés dans les Figures 4 et 5.

On observe que :
- La farine référence 1 est moins sucrée que les 4 lots produits, plus colorée, plus nappante ;
- Les farines 1 et 3 sont toutes les deux les plus sucrées ; le lavage est donc une étape importante pour assurer la neutralité du produit ;
- Les farines 1 et 2 présentent un arrière-goût, ce qui montre l'intérêt du traitement HTST. Sur ces 2 farines, le panel perçoit une aromatique différente, jamais rencontrée auparavant, qualifiée comme suit : acidité du yaourt, « herbale », amertume, chimique, piquant, et ce de façon plus intense/caractéristique sur la farine 1 que sur la farine 2.

En ajoutant une étape de lavage après l'opération de traitement thermique HTST, la neutralité sensorielle de l'échantillon est améliorée avec une réduction de la note sucrée.

La combinaison intégrant les étapes d'HTST puis de lavage de la biomasse avant broyage permet ainsi d'améliorer les propriétés organoleptiques du produit fini en éliminant une note caractéristique de la biomasse « crue », en améliorant sa neutralité et en atténuant la note sucrée.

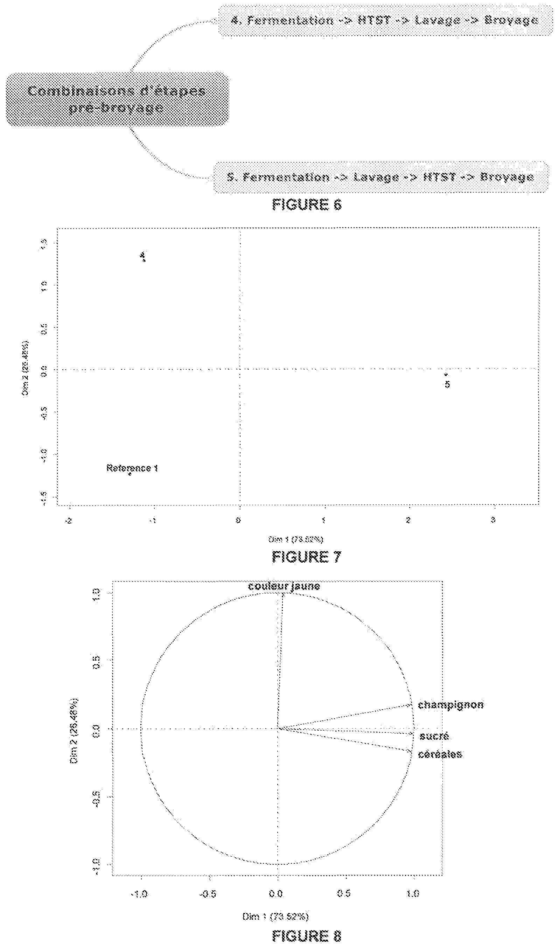

Des combinaisons supplémentaires ont été testées pour affiner la caractérisation de l'impact sensoriel du procédé de « pré-broyage ». Figure 6

Ici, les opérations d'HTST et de Lavage sont inversées :
Combinaison 4 : HTST puis Lavage (même combinaison que ci-dessus)
Combinaison 5 : HTST après Lavage.

Le Tableau IV suivant présente la liste des descripteurs discriminants sur cet ensemble de produits (p-value inférieure à 0,2 sur l'effet Farine):

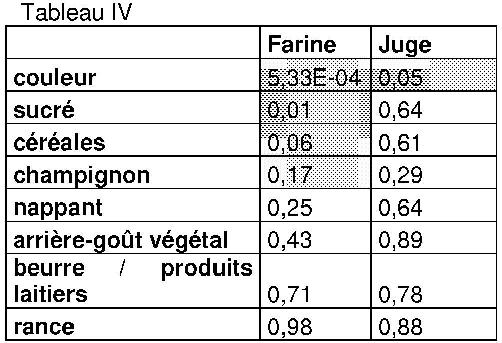

On réalise une Analyse en Composante Principale pour représenter les différences entre les deux différentes farines produites (toujours par rapport au témoin : lot référence 1).

Les résultats sont présentés dans les Figures 7 et 8.

En inversant les deux étapes, la farine de microalgues correspondant au lavage avant HTST (Combinaison 5) présente une aromatique plus intense en champignon / céréales et sucrée.

De plus, les panélistes ont commenté le produit comme étant « piquant ».

La Combinaison 4 est ici préférée pour son profil sensoriel plus neutre et plus favorable en applications alimentaires.

### E. Impact du traitement thermique lui-même sur la qualité de la biomasse

L'opération de traitement thermique provoque une désactivation cellulaire ayant une incidence sur les propriétés de la biomasse.

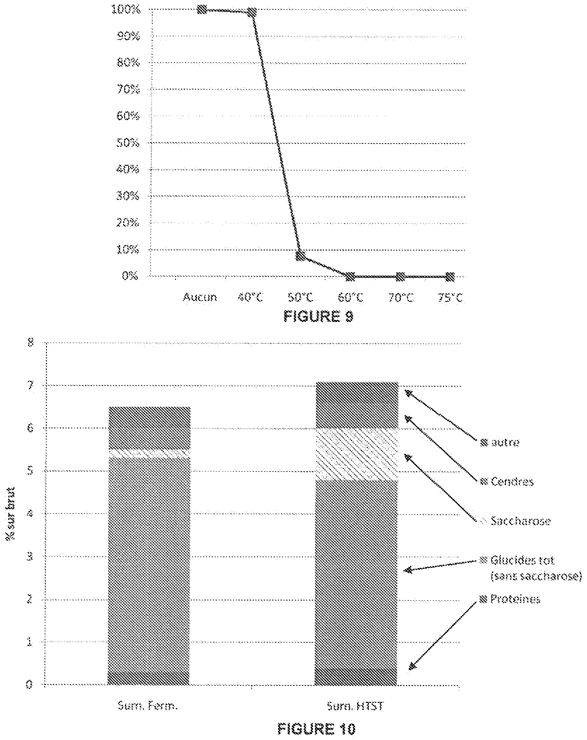

Le pourcentage de désactivation cellulaire (exprimée en % de cellules viables résiduelles après 1 minute de traitement thermique) en fonction des conditions de traitement thermique est présenté sur la figure 9.

Pour un traitement thermique d'une durée de 1 minute, un pourcentage de désactivation supérieur à 90% est atteint à partir de 50°C.

La désactivation cellulaire s'accompagne d'un phénomène de relargage de solubles intracellulaires dans le milieu extracellulaire. Ce phénomène est probablement lié à une perméabilisation pariétale.

Une baisse de la pureté cellulaire, lié à une augmentation de la matière sèche du milieu extracellulaire, est en général observée après traitement thermique de la biomasse (figure 10). Les solubles relargués sont constitués majoritairement de saccharose et, dans une proportion moindre, de sels et protéines.

Un plan d'expérience dans lequel on fait varier les conditions de traitement thermique a été réalisé.

Le tableau suivant présente les lots produits en faisant varier les conditions de traitement thermique (HTST).

| lots | Conditions opératoires |
|---|---|
| 42 | Pas de traitement HTST |
| 23 | HTST 1 min à 75°C |
| 43 | HTST 1 min à 65°C |
| 45 | HTST 1 min à 50°C |
| 46 | HTST 3 min à 95°C |

Le tableau V suivant présente la liste des descripteurs discriminants sur cet ensemble de lots (p-value inférieure à 0,2 sur l'effet produit):

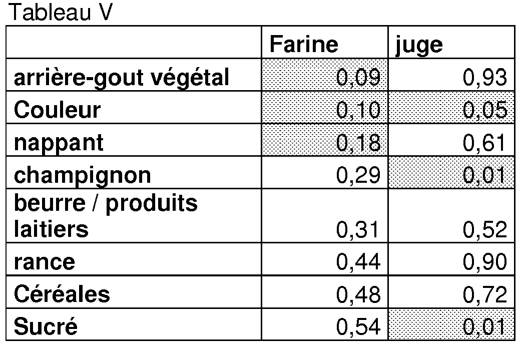

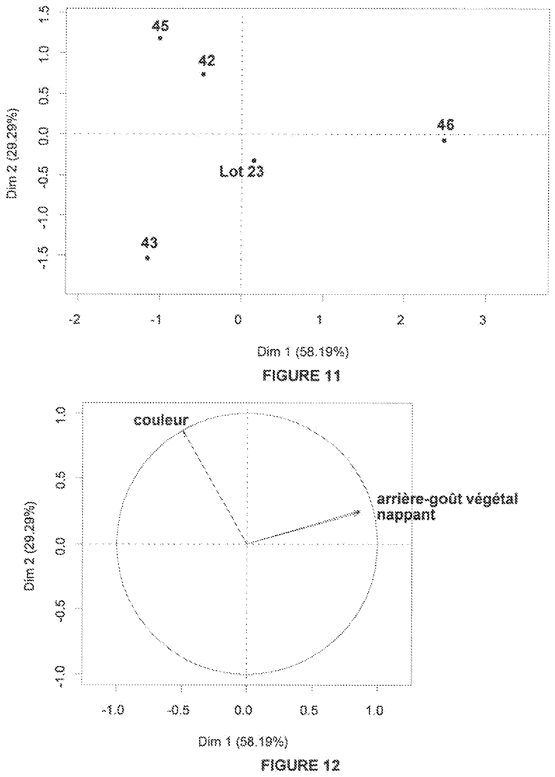

On réalise l'ACP pour représenter les différences entre les différents lots (Figures 11 et 12).

Peu de descripteurs sont discriminants sur cet espace produit car des défauts ont été perçus sur des descripteurs autres que ceux de l'évaluation.

En effet, les lots 42 et 45, en plus d'avoir une couleur plus foncée sont particulièrement amers, piquants, fermentés, laissant une sensation métallique en bouche.

Ces 2 lots sont les moins traités thermiquement (42 n'a pas reçu de traitement HTST et 45 : 1 min à 50°C).

Le lot 46 présente quant à lui un arrière-goût végétal, le traitement thermique de 3 min à 95°C serait donc défavorable à la qualité sensorielle du produit.

Le lot 23 présente un profil intermédiaire, le traitement thermique 1 min / 65°C (lot 43) est le plus favorable pour obtenir un profil sensoriel neutre.

### F. Impact du lavage

De la même façon que précédemment, la société Demanderesse a exploré le couplage de ces nouvelles conditions optimisées de traitement thermique avec une étape de Lavage optimisée, permettant d'entraîner ces solubles extracellulaires, afin d'obtenir des propriétés organoleptiques améliorées des farines de microalgues produites

Différentes conditions de lavage ont été testées.

Le tableau suivant présente les lots produits en faisant varier les conditions de lavage (selon un ratio volume d'eau/ volume de biomasse).

| Lots | Conditions opératoires |
|---|---|
| 47 | Pas de Lavage |
| 49 | Lavage 6/1 (eau/biomasse)) |
| 50 | Lavage 1/1 (eau/biomasse) |
| 51 | Lavage 3/1 (eau/biomasse) |

On notera que ce plan d'expérience permet d'analyser l'impact par lavage « croissant » (du « moins lavé » au « plus lavé », du lot 47 < lot 50 < lot 51 < lot 49).

Le tableau VI suivant présente la liste des descripteurs discriminants sur cet ensemble de lots (p-value inférieure à 0.2 sur l'effet Farine):

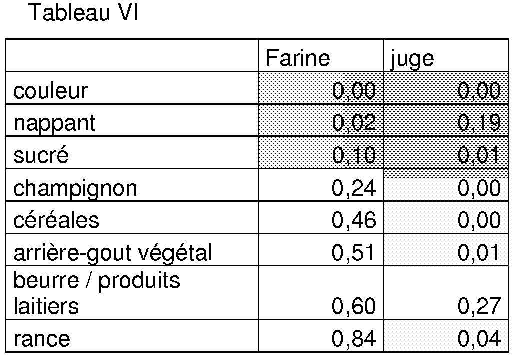

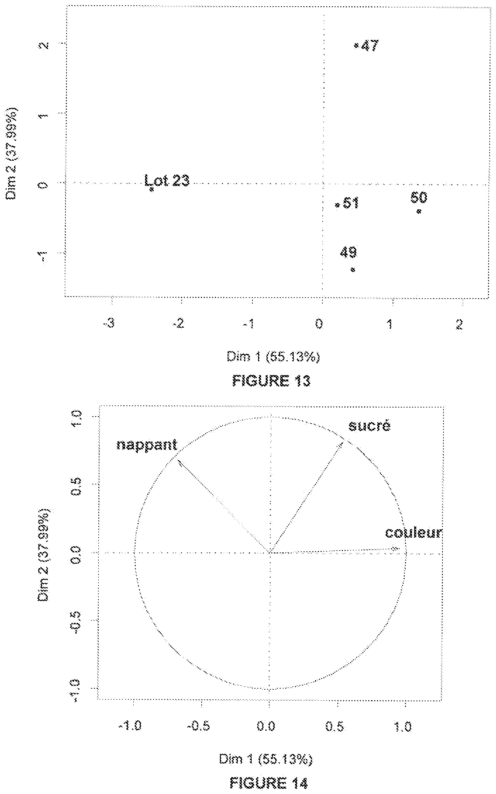

On réalise l'ACP pour représenter les différences entre les différents lots. Les résultats sont représentés dans les Figures 13 et 14.

Cette étude mets bien en évidence le caractère indispensable du lavage. Le produit 47, non lavé, est plus sucré, et a été jugé inacceptable. Le produit non lavé (47) se démarque des autres car plus sucré et présentant un goût différent, atypique.

Les autres produits de cette étude ont un profil sensoriel similaire.

On notera tout de même qu'un lavage « simple » (1 seul volume d'eau par volume de biomasse) entraine une qualité du produit tout à fait convenable, et par la même un gain économique appréciable à l'échelle industrielles (1 volume d'eau utile plutôt que 6 volumes d'eau par volume de biomasse traitée).

### G. Impact de l'acidification lors de la récolte de la biomasse

Un des paramètres qui n'est pas du tout considéré dans le contrôle des étapes responsables de la qualité organoleptique des farines produites est l'incidence du protocole d'arrêt de la fermentation.

Classiquement, lorsqu'en fin de fermentation la valeur de la pO₂ remonte, signe d'une consommation totale du glucose résiduel, le protocole de fin de fermentation est constitué des étapes suivantes :
- Arrêt de la régulation du pH,
- Refroidissement du fermenteur à une Tp < 20°C,
- Réduction de l'agitation et du débit d'air, et
- Maintien d'une pression d'air sur le dôme.

Il se produit généralement une chute progressive du pH de fermentation initial (qu'il soit d'ailleurs fixé à 5,2 ou à 6,8) à un pH proche de 4.

Il a été mis en évidence par la société Demanderesse que cette acidification est corrélée à une sécrétion d'acide lactique issue d'un métabolisme limité en apport d'O₂.

Cette observation a donc été évaluée d'un point de vue sensoriel de manière à mesurer l'impact de la durée de la phase de stockage avant conditionnement de la biomasse, stockage entrainant cette acidification.

Deux lots sont produits : avec stockage pendant une durée de 8 heures (favorisant l'acidification) et sans stockage.

Le tableau VII suivant présente la liste des descripteurs discriminants sur cet ensemble de produits (p-value inférieure à 0.2 sur l'effet Farine).

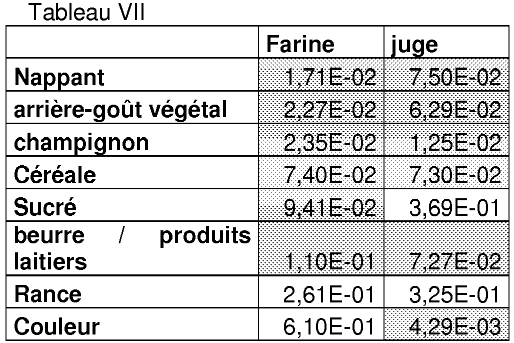

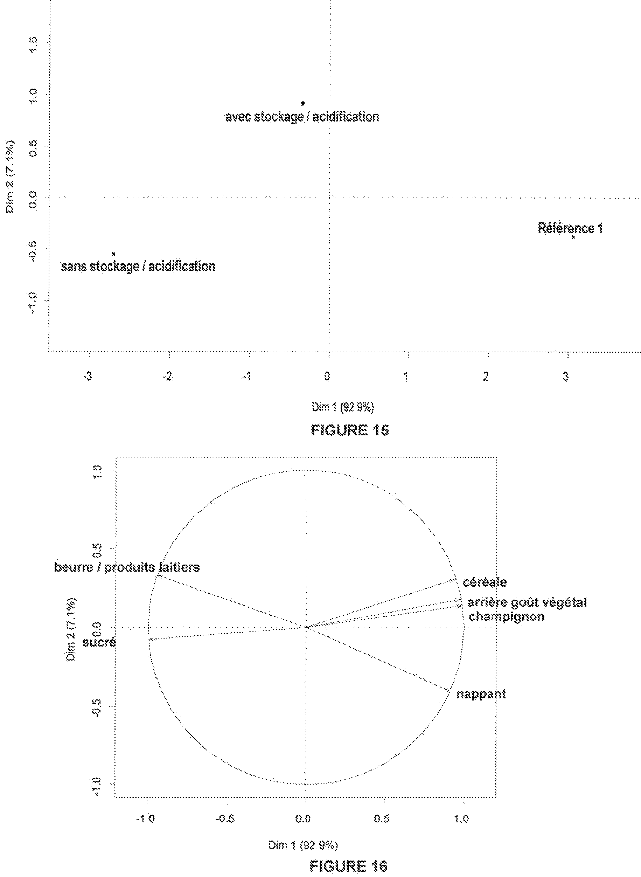

On réalise l'ACP pour représenter les différences entre les farines. Les résultats sont représentés dans les Figures 15 et 16.

Les 3 produits (dont Lot référence 1) se classent sur un axe allant de beurre / produits laitiers / sucré à céréale / champignon / arrière-goût végétal / nappant :
Le lot « sans stockage / acidification » est plus sucré et plus beurre / produits laitiers. La référence 1 est plus nappante avec aromatique céréale / champignon / arrière-goût végétal ; l'essai « avec stockage / acidification » se situe entre les 2.

Si on compare relativement les essais avec ou sans stockage / acidification, l'essai « sans stockage / acidification » est donc plus « neutre », il présente moins de défauts que l'essai « avec stockage / acidification ».

L'analyse sensorielle met donc en évidence qu'une longue phase de stockage couplée à ce phénomène d'acidification dégrade légèrement le profil sensoriel du produit fini puisqu'une note céréale/champignon/ arrière-goût végétal de faible intensité apparait.

### Description des Figures

**FIGURE 1** **:** Représentation graphique des différents lots (nuage de points) de l'ACP - Impact du pH de la fermentation
**FIGURE 2** **:** Cercle de corrélation de l'ACP représentant les profils sensoriels des différents lots - Impact du pH de la fermentation
**FIGURE 3** **:** Combinaisons de pré-broyage testées.
**FIGURE 4** **:** Représentation graphique des différents lots (nuage de points) de l'ACP - impact des étapes de conditionnement de la biomasse avant son broyage
**FIGURE 5** **:** Cercle de corrélation de l'ACP représentant les profils sensoriels des différents lots - impact des étapes de conditionnement de la biomasse avant son broyage
**FIGURE 6** **:** Autres combinaisons de pré-broyage testées
**FIGURE 7** **:** Représentation graphique des différents lots (nuage de points) de l'ACP - impact des étapes de conditionnement de la biomasse avant son broyage
**FIGURE 8** **:** Cercle de corrélation de l'ACP représentant les profils sensoriels des différents lots - impact des étapes de conditionnement de la biomasse avant son broyage
**FIGURE 9** **:** Pourcentage de cellules viables résiduelles après 1 minute de traitement thermique en fonction des conditions de traitement thermique.
**FIGURE 10** **:** Comparaison de la composition de la fraction surnageant extraite de la biomasse avant et après traitement thermique (HTST)
**FIGURE 11** **:** Représentation graphique des différents lots (nuage de points) de l'ACP **-** impact du traitement thermique
**FIGURE 12** **:** Cercle de corrélation de l'ACP représentant les profils sensoriels des différents lots - impact du traitement thermique
**FIGURE 13** **:** Représentation graphique des différents lots (nuage de points) de l'ACP - impact du lavage
**FIGURE 14** **:** Cercle de corrélation de l'ACP représentant les profils sensoriels des différents lots - impact du lavage
**FIGURE 15** **:** Représentation graphique des différents lots (nuage de points) de l'ACP - impact de l'acidification lors de la récolte de la biomasse
**FIGURE 16** **:** Cercle de corrélation de l'ACP représentant les profils sensoriels des différents lots - impact de l'acidification lors de la récolte de la biomasse

## Revendications

1. Procédé de conditionnement d'une biomasse de microalgues du genre *Chlorella*, de préférence *Chlorella protothecoides*, produites en conditions hétérotrophiques et en absence de lumière pour la préparation d'une farine présentant un profil sensoriel optimisé, le procédé de conditionnement étant **caractérisé en ce qu'**il comprend les étapes de :
- collecte de la biomasse directement en fin de fermentation, et stockage pendant moins de 8 heures avant son conditionnement,
- traitement thermique Haute Température / Temps Court (HTST) de la biomasse ainsi récupérée et stockée, pendant 30 secondes à 1 min 30, à une température comprise entre 60 et 68 °C, et
- lavage de la biomasse traitée par HTST avec au maximum 3 volumes d'eau par volume de biomasse.

2. Procédé selon la revendication 1, **caractérisé en ce que** la durée de stockage de la biomasse avant son conditionnement et son broyage est de moins de 3 heures, de préférence de moins de 1 heure.

3. Procédé selon la revendication 1 ou 2, **caractérisé en ce que** le traitement thermique HTST est mené pendant 1 minute à une température de 65°C ± 2 °C, de préférence de 65°C.

4. Procédé selon l'une quelconque des revendications 1 à 3, **caractérisé en ce que** la biomasse est lavée avec un volume d'eau par volume de biomasse.

5. Procédé selon l'une quelconque des revendications 1 à 4, **caractérisé en ce que** le traitement HTST est réalisé avant l'étape de lavage de la biomasse.

6. Procédé selon l'une quelconque des revendications 1 à 5, **caractérisé en ce que** la biomasse a été obtenue avec une fermentation de la microalgue du genre Chlorella à un pH initial entre 6,5 et 7, de préférence 6,8, et avec la régulation du pH de fermentation à une valeur comprise entre 6,5 et 7, de préférence à une valeur de 6,8.

7. Procédé de production d'une biomasse de microalgues du genre *Chlorella*, de préférence *Chlorella protothecoides*, pour la préparation d'une farine présentant un profil sensoriel optimisé, comprenant :
- la production d'une biomasse par fermentation de microalgues du genre *Chlorella*, de préférence *Chlorella protothecoides*, en conditions hétérotrophiques et en l'absence de lumière, le pH initial de la fermentation et la régulation du pH pendant fermentation étant fixée à une valeur comprise entre 6,5 et 7, de préférence à une valeur de 6,8 ; et
- le conditionnement de la biomasse par un procédé selon l'une quelconque des revendications 1 à 5.

8. Procédé de préparation d'une farine de microalgues du genre *Chlorella*, de préférence *Chlorella protothecoides*, présentant un profil sensoriel optimisé, comprenant :
- le conditionnement de la biomasse par un procédé selon l'une quelconque des revendications 1 à 5 ;
- le broyage de la biomasse conditionnée ; et
- le séchage de la biomasse broyée.

9. Procédé de préparation d'une farine de microalgues du genre *Chlorella,* de préférence *Chlorella protothecoides*, présentant un profil sensoriel optimisé, selon la revendication 8, comprenant en outre, préalablement au conditionnement, la production d'une biomasse par fermentation de microalgues du genre *Chlorella*, de préférence *Chlorella protothecoides*, en conditions hétérotrophiques et en l'absence de lumière, le pH initial de la fermentation et la régulation du pH pendant fermentation étant fixée à une valeur comprise entre 6,5 et 7, de préférence à une valeur de 6,8.

## Patentansprüche

1. Verfahren zur Konditionierung einer Biomasse von Mikroalgen der Gattung *Chlorella*, vorzugsweise *Chlorella protothecoides*, produziert unter heterotrophen Bedingungen und unter Ausschluss von Licht zur Herstellung eines Mehls, das ein optimiertes sensorisches Profil aufweist, wobei das Verfahren zur Konditionierung **dadurch gekennzeichnet ist, dass** es die Schritte umfasst:
- Sammlung der Biomasse direkt nach Beendigung der Fermentation und Lagerung weniger als 8 Stunden vor ihrer Konditionierung,
- kurzzeitige thermische Behandlung bei hoher Temperatur (High Temperature / Short Time (HTST)) der auf diese Weise gewonnenen und gelagerten Biomasse von 30 Sekunden bis zu 1 min 30 bei einer Temperatur zwischen 60 und 68°C, und
- Waschen der HTST-behandelten Biomasse mit maximal 3 Volumenteilen Wasser pro Volumenteil Biomasse.

2. Verfahren gemäß Anspruch 1, **dadurch gekennzeichnet, dass** die Dauer der Lagerung der Biomasse vor ihrer Konditionierung und ihrer Zerkleinerung weniger als 3 Stunden, vorzugsweise weniger als 1 Stunde, beträgt.

3. Verfahren gemäß Anspruch 1 oder 2, **dadurch gekennzeichnet, dass** die thermische HTST-Behandlung eine 1 Minute bei einer Temperatur von 65°C ± 2°C, vorzugsweise 65°C, durchgeführt wird.

4. Verfahren gemäß einem der Ansprüche 1 bis 3, **dadurch gekennzeichnet, dass** die Biomasse mit einem Volumenteil Wasser pro Volumenteil Biomasse gewaschen wird.

5. Verfahren gemäß einem der Ansprüche 1 bis 4, **dadurch gekennzeichnet, dass** die HTST-Behandlung vor dem Schritt des Waschens der Biomasse erfolgt.

6. Verfahren gemäß einem der Ansprüche 1 bis 5, **dadurch gekennzeichnet, dass** die Biomasse mit einer Fermentation der Mikroalgen der Gattung *Chlorella* bei einem anfänglichen pH zwischen 6,5 und 7, vorzugsweise 6,8, und mit einer pH-Regulation der Fermentation auf einen Wert zwischen 6,5 und 7, vorzugsweise auf einen Wert von 6,8, erhalten worden ist.

7. Verfahren zur Herstellung einer Biomasse von Mikroalgen der Gattung *Chlorella*, vorzugsweise *Chlorella protothecoides* zur Herstellung eines Mehls, das ein optimiertes sensorisches Profil aufweist, umfassend:
- die Produktion einer Biomasse mittels Fermentation von Mikroalgen der Gattung *Chlorella*, vorzugsweise *Chlorella protothecoides*, unter heterotrophen Bedingungen und unter Ausschluss von Licht, wobei der anfängliche pH der Fermentation und die Regulation des pH während der Fermentation auf einem Wert zwischen 6,5 und 7, vorzugsweise auf einem Wert von 6,8, gehalten wird; und
- die Konditionierung der Biomasse mithilfe eines Verfahrens gemäß einem der Ansprüche 1 bis 5.

8. Verfahren zur Herstellung eines Mehls von Mikroalgen der Gattung *Chlorella*, vorzugsweise *Chlorella protothecoides*, das ein optimiertes sensorisches Profil aufweist, umfassend:
- die Konditionierung der Biomasse mithilfe eines Verfahrens gemäß einem der Ansprüche 1 bis 5;
- die Zerkleinerung der konditionierten Biomasse; und
- die Trocknung der zerkleinerten Biomasse.

9. Verfahren zur Herstellung eines Mehls von Mikroalgen der Gattung *Chlorella*, vorzugsweise *Chlorella protothecoides*, das ein optimiertes sensorisches Profil aufweist, gemäß Anspruch 8, umfassend außerdem vor der Konditionierung die Produktion einer Biomasse mittels Fermentation von Mikroalgen der Gattung *Chlorella*, vorzugsweise *Chlorella protothecoides*, unter heterotrophen Bedingungen und unter Ausschluss von Licht, wobei der anfängliche pH der Fermentation und die Regulation des pH während der Fermentation auf einem Wert zwischen 6,5 und 7, vorzugsweise auf einen Wert von 6,8, gehalten wird.

## Claims

1. A method for conditioning a biomass of microalgae of the *Chlorella* genus, preferably *Chlorella protothecoides*, produced under heterotrophic conditions and in the absence of light for the preparation of a flour having an optimized sensory profile, the conditioning method being **characterized in that** it comprises the steps of:
- collection of the biomass directly at the end of fermentation, and storage for less than 8 hours before conditioning thereof,
- high temperature / short time (HTST) heat treatment of the thus recovered and stored biomass, for 30 seconds to 1 min 30, at a temperature of between 60 and 68°C, and
- washing of the HTST-treated biomass with at most 3 volumes of water per volume of biomass.

2. The method as claimed in claim 1, **characterized in that** the storage time for the biomass before it is conditioned and milled is less than 3 hours, preferably less than 1 hour.

3. The method as claimed in claim 1 or 2, **characterized in that** the HTST heat treatment is carried out for 1 minute at a temperature of 65°C ± 2°C, preferably of 65°C.

4. The method as claimed in any one of claims 1 to 3, **characterized in that** the biomass is washed with one volume of water per volume of biomass.

5. The method as claimed in any one of claims 1 to 4, **characterized in that** the HTST treatment is carried out before the step of washing the biomass.

6. The method as claimed in any one of claims 1 to 5, **characterized in that** the biomass was obtained with fermentation of the microalga of the *Chlorella* genus at an initial pH between 6.5 and 7, preferably 6.8, and with regulation of the fermentation pH at a value of between 6.5 and 7, preferably at a value of 6.8.

7. A method for producing a biomass of microalgae of the *Chlorella* genus, preferably *Chlorella protothecoides*, for the preparation of a flour having an optimized sensory profile, comprising:
- the production of a biomass by fermentation of microalgae of the *Chlorella* genus, preferably *Chlorella protothecoides*, under heterotrophic conditions and in the absence of light, the initial pH of the fermentation and the regulation of the pH during fermentation being fixed at a value of between 6.5 and 7, preferably at a value of 6.8; and
- the conditioning of the biomass by means of a method as claimed in any one of claims 1 to 5.

8. A method for preparing a flour of microalgae of the *Chlorella* genus, preferably *Chlorella protothecoides*, having an optimized sensory profile, comprising:
- the conditioning of the biomass by means of a method as claimed in any one of claims 1 to 5;
- the milling of the conditioned biomass; and
- the drying of the milled biomass.

9. The method for preparing a flour of microalgae of the *Chlorella* genus, preferably *Chlorella protothecoides*, having an optimized sensory profile, as claimed in claim 8, also comprising, prior to the conditioning, the production of a biomass by fermentation of microalgae of the *Chlorella* genus, preferably *Chlorella protothecoides*, under heterotrophic conditions and in the absence of light, the initial pH of the fermentation and the regulation of the pH during fermentation being fixed at a value of between 6.5 and 7, preferably at a value of 6.8.
